(19) Europäisches Patentamt

European Patent Office

Office européen des brevets

(11) **EP 1 707 963 B1**

(12) # EUROPEAN PATENT SPECIFICATION

(45) Date of publication and mention
of the grant of the patent:
**08.12.2010 Bulletin 2010/49**

(51) Int Cl.:
**G01N 33/543** (2006.01)

(21) Application number: **06111999.6**

(22) Date of filing: **30.03.2006**

(54) **Magnetic particles having porous surfaces, method of producing the same, carrier for biochemical use, and biotin-bonding particles**

Magnetische Teilchen mit porösen Oberflächen, Herstellungsverfahren dafür, Träger zur biochemischen Verwendung und Biotin-bindende Teilchen

Particules magnétiques à surface poreuse, procédé de production de celles-ci, support pour utilisation biochimique et particules de liaison biotine

(84) Designated Contracting States:
**CH DE FR GB LI**

(30) Priority: **31.03.2005 JP 2005102435**
**24.05.2005 JP 2005151115**

(43) Date of publication of application:
**04.10.2006 Bulletin 2006/40**

(73) Proprietor: **JSR Corporation**
**Minato-ku**
**Tokyo 105-8640 (JP)**

(72) Inventors:
• **Tamori, Kouji**
**Tokyo 105-8640 (JP)**
• **Ueno, Masaru**
**Tokyo 105-8640 (JP)**

(74) Representative: **TBK-Patent**
**Bavariaring 4-6**
**80336 München (DE)**

(56) References cited:
**EP-A- 1 020 488     US-A- 5 814 687**

• **NISHIBIRAKI H ET AL: "Preparation of medical magnetic nanobeads with ferrite particles encapsulated in a polyglycidyl methacrylate (GMA) for bioscreening" JOURNAL OF APPLIED PHYSICS, AMERICAN INSTITUTE OF PHYSICS. NEW YORK, US, vol. 97, no. 10, 17 May 2005 (2005-05-17), pages 10Q919-10Q919, XP012070142 ISSN: 0021-8979**
• **SPANOVA ALENA ET AL: "Immunomagnetic separation and detection of Salmonella cells using newly designed carriers." JOURNAL OF CHROMATOGRAPHY A, vol. 1009, no. 1-2, 15 August 2003 (2003-08-15), pages 215-221, XP002394442 ISSN: 0021-9673**
• **SPANOVA ALENA ET AL: "Magnetic hydrophilic methacrylate-based polymer microspheres designed for polymerase chain reactions applications." JOURNAL OF CHROMATOGRAPHY B, vol. 800, no. 1-2, 5 February 2004 (2004-02-05), pages 27-32, XP002394451 ISSN: 1570-0232**
• **KRIZOVA JANA ET AL: "Magnetic hydrophilic methacrylate-based polymer microspheres for genomic DNA isolation" JOURNAL OF CHROMATOGRAPHY A, vol. 1064, no. 2, 4 February 2005 (2005-02-04), pages 247-253, XP002394443 ISSN: 0021-9673**

**Description**

BACKGROUND OF THE INVENTION

The present invention relates to magnetic particles excelling in magnetic separating capability and capability of binding a large amount of biochemical substances, a method of producing the same, a carrier for biochemical use, and biotin-binding particles.

Magnetic particles with a small average particle diameter can provide an excellent field for antigen-antibody immunoreaction, hybridization of DNAs, or hybridization of DNA and RNA due to the large surface area per unit weight. For this reason, application of magnetic particles particularly to research into diagnostic agents and medicines has been actively promoted.

It is possible to increase the amount of biochemical substances bonded to the surfaces of magnetic particles by providing a large surface area by increasing porosity of the surfaces. USP No. 4,774,265, for example, discloses magnetic particles having porous surfaces obtained by producing a magnetic substance from iron ions in porous particles. However, this method has the following drawbacks. (1) Since the amount of the magnetic substance produced in particles is limited, magnetic separating capability of the resulting magnetic particles is poor. (2) Since the magnetic substance is also produced outside of particles, a complicated process is required for removing such a magnetic substance produced outside of particles. (3) Even after removing the magnetic substance from outside of the particles, the magnetic substance in the particles maintains contact with a dispersing medium. If these particles are covered with a polymer, the porosity on the surfaces is impaired, resulting in poor capability of binding biochemical substances.

JP-A-5-197212 discloses magnetic particles having irregular surfaces obtained by suspension polymerization of polyfunctional monomers containing a magnetic substance and a solvent, followed by removal of the solvent. JP-B-7-27279 discloses porous magnetic particles formed using a foaming agent. However, the surface porosity of the magnetic particles obtained by these methods is insufficient to exhibit adequate capability of binding a large amount of biochemical substances. US 5814687 describes magnetic polymeric particles polymerized with different monomers.

SUMMARY

The present invention may provide magnetic particles having porous surfaces and exhibiting superior magnetic separating capability and excellent capability of binding a large amount of biochemical substances, a method of producing the magnetic particles, a biochemical carrier, and biotin-binding particles.

The inventor of the present invention has found that magnetic particles having porous surfaces obtained by forming a polymer part (B) soluble in an organic solvent (S) and a polymer part (C) insoluble in the organic solvent (S) on the surfaces of magnetic matrix particles (A) and causing the polymer part (B) to come into contact with the organic solvent (S) exhibit superior magnetic separating capability and have capability of binding a large amount of biochemical substances. This finding has led to the completion of the invention.

According to a first aspect of the invention, there is provided magnetic particles having porous surfaces, the magnetic particles being obtained by forming a polymer part (B) soluble in an organic solvent (S) and a polymer part (C) insoluble in the organic solvent (S) on surfaces of magnetic matrix particles (A) and causing the polymer part (B) to come into contact with the organic solvent (S). There are no limitations to the order of forming the polymer part (B) and polymer part (C). Specifically, forming the polymer part (B) and the polymer part (C) on the surfaces of the magnetic matrix particles (A) includes: forming the polymer part (C) after forming the polymer part (B) on the surfaces of the magnetic matrix particles (A); and simultaneously forming the polymer part (B) and the polymer part (C) on the surfaces of the magnetic matrix particles (A).

In this magnetic particles having porous surfaces,
the polymer part (B) may be formed by radical polymerization of a mono-functional monomer (b) in an aqueous medium in the presence of the magnetic matrix particles (A).

In this magnetic particles having porous surfaces,
the polymer part (C) may be formed by radical polymerization of a poly-functional monomer (c) in an aqueous medium in the presence of the magnetic matrix particles (A).

In this magnetic particles having porous surfaces, the magnetic matrix particles

(A) may contain:

non-magnetic nuclear particles ($A_1$); and
magnetic fine particles ($A_2$) located on the surfaces of the non-magnetic nuclear particles ($A_1$).

In this magnetic particles having porous surfaces,
the magnetic matrix particles (A) may further contain a matrix particle coating layer ($A_3$) which covers the non-magnetic

nuclear particles (A$_1$) and the magnetic fine particles (A$_2$).

According to a second aspect of the invention, there is provided a carrier for use with a biochemical substance, comprising the above-described magnetic particles having porous surfaces.

According to a third aspect of the invention, there is provided biotin-binding particles, comprising a substance having biotin binding sites immobilized on the surfaces of the above-described magnetic particles having porous surfaces.

According to a fourth aspect of the invention, there is provided a method of producing magnetic particles having porous surfaces, comprising:

forming a polymer part (B) soluble in an organic solvent (S) and a polymer part (C) insoluble in the organic solvent (S) on surfaces of magnetic matrix particles (A); and
causing the polymer part (B) to come into contact with the organic solvent (S).

In this method of preparing the magnetic particles having porous surfaces,
the step of forming the polymer part (B) may include radically polymerizing a mono-functional monomer (b) in an aqueous medium in the presence of the magnetic matrix particles (A).

In this method of preparing the magnetic particles having porous surfaces,
the step of forming the polymer part (C) may include radically polymerizing a poly-functional monomer (c) in an aqueous medium in the presence of the magnetic matrix particles (A).

Since the above-described magnetic particles having the porous surfaces have outstanding magnetic separation capability and an extremely large unit surface area, the magnetic particles have superior capability of binding biochemical substances. The above-described magnetic particles can be used as a biochemical carrier, for example, as a carrier for diagnostics, and in a wide variety of fields such as coating materials, paper, electronic industry materials, electrophotography, cosmetics, medical supplies, agricultural chemicals, foods, and catalysts. As an example of the application, the magnetic particles can be used as a diagnostic agent for medical treatment, particularly as particles used with an automatic measuring apparatus.

Magnetic particles having an extremely large unit surface area can be efficiently produced by the above-mentioned method of producing magnetic particles having porous surfaces of the.

BRIEF DESCRIPTION OF THE SEVERAL VIEWS OF THE DRAWING

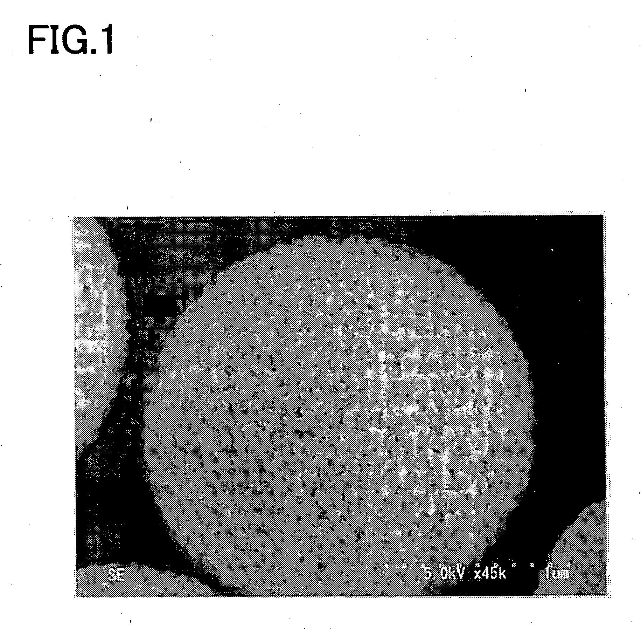

Figure 1 shows an SEM photograph of magnetic particles having porous surfaces of Example 1.

DETAILED DESCRIPTION OF THE EMBODIMENT

The above-described magnetic particles having porous surfaces, method of producing the same, carrier for biochemical use, and biotin-bonding particles will be described in detail below.

1. Magnetic particles having porous surfaces and method of producing the same

The magnetic particles having porous surfaces (hereinafter referred to from time to time simply as "magnetic particles") of one embodiment of the invention can be obtained by forming a polymer part (B) soluble in an organic solvent (S) and a polymer part (C) insoluble in the organic solvent (S) on the surfaces of magnetic matrix particles (A) and causing the polymer part (B) to come into contact with the organic solvent (S). The materials and the method used for producing the magnetic particles of this embodiment will be described below in detail.

The diameter of the magnetic particles having porous surfaces of this embodiment is preferably from 0.1 to 10 $\mu$m, and more preferably from 0.5 to 5.0 $\mu$m. If the diameter of the particles is less than 0.1 $\mu$m, a sufficient magnetic response cannot be exhibited. It takes a considerably long time for separating the particles or a considerably large magnetic force is required for the separation. On the other hand, if the diameter of the particles is more than 10.0 $\mu$m, the particles easily sediment in a dispersion medium. It is therefore necessary to stir the dispersion medium to cause the particles to bind with the target substance. In addition, the proportion of the surface area to the weight of the particles decreases, possibly making it more difficult for the particles to combine with a sufficient amount of the target substance.

The magnetic particles having porous surfaces of this embodiment can be used dispersed in a dispersion medium. As the dispersion medium, an aqueous medium can be given, for example. Examples of the aqueous medium include, but are not limited to, water and aqueous solvents. As examples of the aqueous solvent, alcohols (for example, ethanol, alkylene glycol, mono alkyl ether, etc.) can be given.

1.1 Magnetic matrix particles (A)

The magnetic matrix particles (A) are known particulate substances that can be magnetically collected, having a particle diameter (d) of preferably from 0.1 to 10 $\mu$m, more preferably from 0.2 to 7 $\mu$m, and most preferably from 0.5 to 5.0 $\mu$m. If d is less than 0.1 $\mu$m, it may take a long time for separation using a magnetic force; if more than 10 $\mu$m, the amount of biochemical substances that can bond to the particles may be small.

The magnetic matrix particles (A) may have a homogeneous internal composition. However, since many homogeneous magnetic matrix particles (A) with a particle size in the above-described preferable range are paramagnetic, re-dispersion may become difficult if a separation-purification operation using a magnetic force is repeated. For this reason, the magnetic matrix particles (A) more preferably have a heterogeneous internal composition that contains magnetic fine particles with least residual magnetization. As the internal structure of the magnetic matrix particles (A) having such a heterogeneous internal composition, a structure containing magnetic fine particles dispersed in a continuous phase of non-magnetic material such as a polymer, a structure having a secondary aggregate of magnetic fine particles as a core and a non-magnetic material such as a polymer as a shell, a structure having a non-magnetic material such as a polymer as a core and a secondary aggregate of magnetic fine particles as a shell, and the like can be given.

Of these, the structure having a non-magnetic material such as a polymer as a core and a secondary aggregate of magnetic fine particles as a shell is a preferable internal structure of the magnetic matrix particles (A). In this case, the magnetic matrix particles (A) may contain non-magnetic nuclear particles ($A_1$) and magnetic fine particles ($A_2$) located on the surfaces of the non-magnetic nuclear particles ($A_1$). In this case, the magnetic matrix particles (A) can be produced by causing magnetic fine particles ($A_2$) to be physically adsorbed on the surfaces of the non-magnetic nuclear particles ($A_1$) as later described in more detail. In this case, the magnetic matrix particles (A) may further contain a matrix particle coating layer ($A_3$) which covers the non-magnetic nuclear particles ($A_1$) and the magnetic fine particles ($A_2$), if necessary. The magnetic matrix particles (A) may further contain a matrix particle coating layer ($A_3$) which covers the non-magnetic nuclear particles ($A_1$) and the magnetic fine particles ($A_2$) (later described in more detail).

1.1.1 Non-magnetic nuclear particles ($A_1$)

Although either an organic substance or an inorganic substance can be used as the non-magnetic nuclear particles ($A_1$), an organic substance is more preferable. The following polymers can be given as examples of the organic substances. Particularly preferable polymers are vinyl polymers prepared from aromatic vinyl monomers such as styrene, alpha-methylstyrene, halogenated styrene, and divinylbenzene; vinyl esters such as vinyl acetate and vinyl propionate; unsaturated nitriles such as acrylonitrile; ethylenically unsaturated carboxylic acid alkyl esters such as methyl acrylate, ethyl acrylate, ethyl methacrylate, butyl acrylate, butyl methacrylate, 2-ethylhexyl acrylate, 2-ethylhexyl methacrylate, lauryl acrylate, lauryl methacrylate, ethylene glycol diacrylate, ethylene glycol dimethacrylate, cyclohexyl acrylate, and cyclohexyl methacrylate; and the like. The vinyl polymer may be either a homopolymer or a copolymer of two or more monomers selected from the above-mentioned vinyl monomers. Copolymers of the above-mentioned vinyl monomers and copolymerizable monomers such as a conjugated diolefin (e.g. butadiene, isoprene), acrylic acid, methacrylic acid, acrylamide, methacrylamide, glycidyl acrylate, glycidyl methacrylate, N-methylolacrylamide, N-methylolmethacrylamide, 2-hydroxyethyl acrylate, 2-hydroxyethyl methacrylate, diallyl phthalate, allyl acrylate, allyl methacrylate, trimethylolpropane triacylate, trimethylolpropane trimethacrylate, and the like may also be used.

The non-magnetic nuclear particles ($A_1$) used in this embodiment have an average particle diameter of preferably from 0.1 to 10 $\mu$m, more preferably from 0.2 to 5.0 $\mu$m, and most preferably from 0.3 to 3.0 $\mu$m.

The polymer particles which can be used as the non-magnetic nuclear particles ($A_1$) having an average particle diameter of the above range can be obtained by the suspension polymerization of the above-mentioned vinyl monomers or by grinding a bulk polymer. The non-magnetic nuclear particles ($A_1$) having a uniform particle diameter can be easily produced by the swelling polymerization method described in JP-B-57-24369, the polymerization method described in J. Polym. Sci., Polymer Letter Ed. 21, 937 (1963), methods described in JP-A-61-215602, JP-A-61-215603, JP-A-61-215604, and the like.

1.1.2 Magnetic fine particles ($A_2$)

There are no specific limitations on the type of the magnetic fine particles ($A_2$). Typical examples are iron oxides, which include ferrite represented by $MFe_2O_4$ (wherein M is Co, Ni, Mg, Cu, $Li_{0.5}Fe_{0.5}$, etc.), magnetite represented by $Fe_3O_4$, and gamma-$Fe_2O_3$. Gamma-$Fe_2O_3$ and $Fe_3O_4$ are particularly preferable magnetic materials with high saturated magnetization and low residual magnetization.

The magnetic fine particles ($A_2$) used in this embodiment have an average particle diameter of preferably 1/5 or less, more preferably 1/10 or less, and still more preferably 1/20 or less, of the average particle diameter of the non-magnetic nuclear particles ($A_1$). If the average particle diameter of the magnetic fine particles ($A_2$) is more than 1/5 of the average particle diameter of non-magnetic nuclear particles $(A_1)$, it may be difficult to form a covering layer of the magnetic fine particles $(A_2)$ on the surfaces of the non-magnetic nuclear particles $(A_1)$ having a uniform and sufficient thickness. From the viewpoint of re-dispersibility after the magnetic separation-purification operation, the magnetic fine particles $(A_2)$ are required to exhibit least residual magnetization. For example, fine particles of ferrite and/or magnetite with a particle size of about 5 to 20 nm can be suitably used.

The magnetic fine particles $(A_2)$ of which the surfaces are hydrophobized may be used. There are no specific limitations to the method of hydrophobizing the surfaces of the magnetic fine particles $(A_2)$. For example, a method of causing a compound having a part with a very high affinity with the magnetic fine particles $(A_2)$ and a hydrophobic part in the molecule to come into contact with and bond to the magnetic fine particles $(A_2)$ can be given. As examples of such a compound, silane compounds represented by a silane coupling agent and a surfactant represented by a long-chain fatty acid soap can be mentioned.

Hydrophobization using a silane compound has an advantage of improving chemical resistance, particularly alkali resistance, and can effectively prevent detachment of magnetic material or a decrease in the magnetic performance due to dropout of the hydrophobic parts, and also invasion of contaminants due to suspension of detached magnetic fine particles $(A_2)$ and surfactants in the system. In this embodiment, the surfaces of magnetic fine particles $(A_2)$ can be judged to be sufficiently hydrophobic when particles are excellently dispersed in toluene, for example.

The ratio by weight of the non-magnetic nuclear particles $(A_1)$ to the magnetic fine particles $(A_2)$ in this embodiment is preferably from 75:25 to 20:80. If the ratio of the magnetic fine particles $(A_2)$ is smaller than this range, the magnetic separation capability may be inferior; if larger than this range, the amount of the magnetic fine particles $(A_2)$ to the non-magnetic nuclear particles $(A_1)$ is excessive, resulting in a tendency of leaving a large amount of magnetic fine particles $(A_2)$ without being complexed.

1.1.3 Production of magnetic matrix particles (A)

In the production of the magnetic matrix particles (A) in this embodiment, in order to locate the magnetic fine particles $(A_2)$ on the surfaces of the non-magnetic nuclear particles $(A_1)$, the non-magnetic nuclear particles $(A_1)$ are mixed with the magnetic fine particles $(A_2)$ to cause the magnetic fine particles $(A_1)$ to be physically adsorbed on the surfaces of the non-magnetic nuclear particles $(A_2)$.

In this embodiment, "to physically adsorb" or "physical adsorption" means adsorption not involving a chemical reaction. As the principle of "physical adsorption," hydrophobic/hydrophobic adsorption, molten bonding or adsorption, fusion bonding or adsorption, hydrogen bonding, van der Waals binding, and the like can be given, for example. As examples of the adsorption utilizing the hydrophobic/hydrophobic adsorption, a method of selecting non-magnetic nuclear particles $(A_1)$ and magnetic fine particles $(A_2)$, both having hydrophobic or hydrophobized surfaces, and dry-blending the non-magnetic nuclear particles $(A_1)$ and the magnetic fine particles $(A_2)$ or sufficiently dispersing the non-magnetic nuclear particles $(A_1)$ and magnetic fine particles $(A_2)$ in a solvent with high dispersibility (e.g. toluene, hexane) without causing one to invade the other, followed by vaporizing the solvent while mixing the two dispersions can be given.

A method of complexing the non-magnetic nuclear particles $(A_1)$ and magnetic fine particles $(A_2)$ by physically applying a strong force from outside is also effective. As a method of physically applying a strong force, a method of using a mortar, automatic mortar, or ball mill, a blade pressing powder compression method, a method of using a mechanochemical effect such as a mechanofusion method, a method of causing collision in a high speed air stream such as a jet mill and hybridizer, and the like can be given. A strong physical adsorption force is desirable for efficient and firm complexing- As a specific method, stirring using a vessel equipped with stirring blades at a stirring blade peripheral velocity of preferably 15 m/sec or more, more preferably 30 m/sec or more, and still more preferably from 40 to 150 m/sec or more can be given. If the stirring blade peripheral velocity is less than 15 m/sec, sufficient energy may not be obtained for causing magnetic fine particles $(A_2)$ to be adsorbed on the surfaces of the non-magnetic nuclear particles $(A_1)$. Although there are no specific limitations, the upper limit of the stirring blade peripheral velocity is determined taking the device used, energy efficiency, and the like into consideration.

1.2 Organic solvent (S)

The organic solvent (S) dissolves the polymer part (B), but does not dissolve the polymer part (C). "Dissolve a polymer" in this embodiment refers to the capability of an organic solvent which is confirmed by putting one g of ground polymer in 100 g of the organic solvent and stirring for 24 hours. If the polymer is not observed by the naked eye, the solvent is judged to be capable of dissolving the polymer. If the polymer is observed by the naked eye, the solvent is judged to be incapable of dissolving the polymer.

As examples of the organic solvent (S), hydrocarbon solvents such as pentane, hexane, cyclohexane, petroleum ether, petroleum benzine, isooctane, octane, benzene, toluene, and xylene; halogenated compounds such as chloroform, 1,2-dichloroethane, 1,2.-dibromoethane, 1,1,2-trichloro-1,2,2-trifluoroethane, chlorobenzene, bromobenzene, and o-dichlorobenzene; alcohols such as ethanol, 1-propanol, 2-propanol, 2,2,2-trifluoroethanol, 1-butanol, 2-butanol, isobutyl alcohol, isopentyl alcohol, cyclohexanol, propylene glycol, and benzyl alcohol; ethers such as diethyl ether and diisopropyl ether; ketones such as acetone and methyl ethyl ketone; and the like can be given. These solvents may be used either alone or mixed. A solvent having an SP value close to the extracted polymer is preferred.

1.3 Formation of polymer part (B)

The polymer part (B) comprises a polymer soluble in the organic solvent (S). Any polymer soluble in the organic solvent (S) can be used as the polymer part (B).

The polymer part (B) can be obtained by radical polymerization of a mono-functional monomer (b) in an aqueous medium, preferably in the presence of the magnetic matrix particles (A). The aqueous media mentioned above as the dispersion media for magnetic particles can be used as the aqueous media here. A preferable aqueous medium is water.

Vinyl monomers are preferable mono-functional monomers (b). Examples which can be given include aromatic vinyl monomers such as styrene, alpha-methylstyrene, halogenated styrene; vinyl esters such as vinyl acetate and vinyl propionate; unsaturated nitriles such as acrylonitrile; ethylenically unsaturated carboxylic acid alkyl esters such as methyl acrylate, ethyl acrylate, methyl methacrylate, ethyl methacrylate, butyl acrylate, butyl methacrylate, 2-ethylhexyl acrylate, 2-ethylhexyl methacrylate, lauryl acrylate, lauryl methacrylate, cyclohexyl acrylate, and cyclohexyl methacrylate; and the like. The polymer part (B) in this embodiment may be either a homopolymer or a copolymer of two or more monomers selected from the above-mentioned monomers (b).

As the polymer part (B), copolymers of the above-mentioned vinyl monomers and copolymerizable monomers such as a conjugated diolefin (e.g. butadiene, isoprene, etc.), acrylic acid, methacrylic acid, acrylamide, methacrylamide, glycidyl acrylate, glycidyl methacrylate, N-methylolacrylamide, N-methylolmethacrylamide, 2-hydroxyethyl acrylate, 2-hydroxyethyl methacrylate, diallyl phthalate, allyl acrylate, allyl methacrylate, trimethylolpropane triacylate, trimethylolpropane trimethacrylate, styrenesulfonic acid and its sodium salt, 2-acrylamide-2-methylpropanesulfonic acid and its sodium salt, isoprenesulfonic acid and its sodium salt, and the like may also be used.

The mono-functional monomer (b) is used in an amount of preferably from 1 to 300 parts by weight, more preferably from 5 to 200 parts by weight, and most preferably from 10 to 150 parts by weight for 100 parts by weight of the magnetic matrix particles (A).

A chain transfer agent is preferably used in polymerizing the mono-functional monomer (b). As examples of the chain transfer agent, allyl alcohol and its esters; thioacrylic acid esters; dienes such as 2,6-dimethyl-2,6-octadiene; alkyl halides such as chloroform, bromoform, carbon tetrachloride, and carbon tetrabromide; aromatic compounds such as toluene and alpha-methylstyrene; aromatic halogenated compounds such as (di)chlorobenzene, (di)bromobenzene, (di)chlorotoluene, (di)bromotoluene, and trichlorotoluene; nitroalkyls such as nitromethane, nitroethane, and trinitromethane; aromatic nitro compounds such as trinitrobenzene, trinitrotoluene, nitrophenol, and trinitrophenol; alkylamines such as trimethylamine and triethylamine; aromatic amines such as aniline, N,N-dimethylaniline, toluidine, and N,N-dimethyl-p-toluidine; metal alkyl compounds such as diethylzinc, triethylaluminum, tetrabutyltin,; metal chlorides such as copper chloride (II), iron chloride (II), and iron chloride (III); phosphorus-containing compounds such as phosphorus trichloride and triethylphosphine; alkyl (di)sulfide such as ethyl (di)sulfide and butyl (di)sulfide; bis(dimethylthiocarbamoyl) sulfides, bis(diethylthiocarbamoyl) sulfides, and bis(dialkylthiocarbamoyl) disulfides; aromatic (di)sulfides such as phenyl (di)sulfide and benzyl (di)sulfide; aromatic (di)sulfides such as benzyl (di)sulfide; alkyl thioalcohols and aromatic thioalcohols such as ethanethiol, propanethiol, benzenethiol, toluenethiol, and tert-dodecanethiol; alkylthiocarboxylic acids, aromatic thiocarboxylic acids, and their esters such as thioacetic acid, ethyl thioacetate, thiobenzoic acid, and ethyl thiobenzoate; sulfoxides such as dimethyl sulfoxide, diphenyl sulfoxide, and dibenzyl sulfoxide; sulfones such as a dimethyl sulfone and diphenyl sulfone; and the like can be given.

Although there are no specific limitations to the chain transfer agents inasmuch as the compound has chain transfer capability, hydrophobic chain transfer agents are preferable. When a hydrophobic chain transfer agent is used, hydrophobic monomers become easily reactive, resulting in polymers with a low molecular weight. The amount of the chain transfer agent used is preferably from 1/1,000 to 1/25 part by weight for 100 parts by weight of the mono-functional monomers.

A polymerization initiator and an emulsifier are preferably used in polymerizing the mono-functional monomer (b).

As the polymerization initiator, an oil soluble initiator and water soluble initiator may be used.

As examples of the oil soluble initiator, peroxides and azo compounds such as benzoyl peroxide, lauroyl peroxide, tert-butyl peroxy-2-ethylhexanate, 3,5,5-trimethylhexanoyl peroxide, azobisisobutylonitrile, and the like can be given.

As examples of the water soluble initiator, persulfates such as potassium persulfate, ammonium persulfate, and sodium persulfate; hydrogen peroxide; mineral acid salt of 2,2-azobis(2-aminopropane); azobiscyanovaleric acid and alkali metal salt or ammonium salt thereof; redox initiation agents consisting of a combination of a persulfate or hydrogen peroxide salt with sodium bisulfite, sodium thiosulfate, or ferrous chloride; and the like can be given. Of these, persulfates are preferably used. The proportion of these initiators to the total amount of monomers is preferably from 0.01 to 8 wt%.

From the viewpoint of water solubility, the oil soluble initiator is more preferable. When a water soluble initiator is used, there is a tendency of producing a large amount of new particles in which only hydrophobic monomers not containing the magnetic matrix particles (A) are polymerized, rather than polymerization on the surfaces of the magnetic matrix particles (A).

As the emulsifier, commonly used anionic surfactants, cationic surfactants, or nonionic surfactants can be used either individually or in combination. Examples of the anionic surfactant include reactive anionic surfactants such as an alkali metal salt of higher alcohol sulfate ester, an alkali metal salt of alkylbenzenesulfonic acid, an alkali metal salt of dialkyl succinate sulfonic acid, an alkali metal salt of alkyl diphenyl ether disulfonic acid, a sulfate of polyoxyethylene alkyl (or alkyl phenyl) ether, a phosphate of polyoxyethylene alkyl (or alkyl phenyl) ether, a formalin condensate of sodium naphthalenesulfonate, commercially available products such as LATEMUL S-180A (manufactured by Kao Corp.), ELEMINOL JS-2 (manufactured by Sanyo Chemical Industries, Ltd.), AQUALON HS-10, KH-10 (manufactured by Daiichi Kogyo Seiyaku Co., Ltd.), Adekareasoap SE-10N, SR-10 (manufactured by Asahi Denka Kogyo K-K.), and the like.

As examples of the cationic surfactant, an alkylamine (salt), polyoxyethylenealkylamine (salt), quaternary alkylammonium salt, alkylpyridinium salt, and the like can be given.

Examples of nonionic surfactant include reactive nonionic surfactants such as polyoxyethylene alkyl ether, polyoxyethylene alkyl phenyl ether, commercially available products such as AQUALON RS-20 (manufactured by Daiichi Kogyo Seiyaku Co., Ltd.) and Adekareasoap NE-20, RN-20 (manufactured by Asahi Denka Kogyo K.K.), and the like.

There are no specific limitations to the method of adding monomers to the polymerization system during formation of polymer layers. All monomers may be added initially, or monomers may be added intermittently or continuously. The polymerization temperature may vary according to the type of initiator, but usually from 10 to 90°C, and preferably from 30 to 85°C. The time required for the polymerization is usually from 1 to 30 hours.

### 1.4 Formation of polymer part (C)

The polymer part (C) comprises a polymer not soluble in the organic solvent (S). Any polymer not soluble in the organic solvent (S) can be used as the polymer part (C).

The polymer part (C) is preferably present together with the polymer part (B) on the surfaces of the magnetic matrix particles (A). Specifically, the polymer part (B) and polymer part (C) are caused to be present together on the surfaces of the magnetic matrix particles (A) by simultaneously forming the polymer part (B) and polymer part (C) on the surfaces of the magnetic matrix particles (A) or by forming the polymer part (C) after forming the polymer part (B).

The polymer part (C) can be obtained by radical polymerization of a poly-functional monomer (c) preferably in an aqueous medium. The aqueous media mentioned above as the dispersion media of magnetic particles can be used as the aqueous media here. A preferable aqueous medium is water.

As examples of the poly-functional monomer (c), diacrylate compounds such as polyethylene glycol diacrylate, diethylene glycol diacrylate, 1,3-butylene glycol diacrylate, polytetramethylene glycol diacrylate, poly(ethylene glycol tetramethylene glycol) diacrylate, poly(propylene glycol tetramethylene glycol) diacrylate, polyethylene glycol polypropylene glycol polyethylene glycol diacrylate, propylene oxide-modified bisphenol A diacrylate, propylene oxide-modified bisphenol A diacrylate, ethylene oxide propylene oxide-modified bisphenol A diacrylate, 1,6-butylene glycol diacrylate, 1,6-hexylene glycol diacrylate, neopentyl glycol diacrylate, polypropylene diacrylate, 2,2'-bis(4-acryloxypropyloxyphenyl) propane, and 2,2'-bis(4-acryloxydiethoxyphenyl)propane; triacylate compounds such as trimethylolpropane triacylate, trimethylolethane triacylate, and tetramethylolmethane triacylate; tetraacrylate compounds such as ditrimethylolpropane tetraacrylate, tetramethylolmethane tetraacrylate, and pentaerythritol tetraacrylate; methacrylate compounds such as ethylene glycol dimethacrylate, glycerol dimethacrylate, diethylene glycol dimethacrylate, 2-hydroxy-3-acryloyloxypropyl methacrylate, triethylene glycol dimethacrylate, polytetramethylene glycol dimethacrylate, polyethylene glycol dimethacrylate, poly(ethylene glycol tetramethylene glycol) dimethacrylate, poly(propylene glycol tetramethylene- glycol) dimethacrylate, polyethylene glycol polypropylene glycol polyethylene glycol dimethacrylate, ethylene oxide-modified bisphenol A dimethacrylate, propylene oxide-modified bisphenol A dimethacrylate, ethylene oxide-propylene oxide-modified bisphenol A dimethacrylate, propylene oxide-ethylene oxide (block-type) modified bisphenol A dimethacrylate, 1,3-butylene glycol dimethacrylate, 1,4-butylene glycol dimethacrylate, 1,6-hexane glycol dimethacrylate, neopentyl glycol dimethacrylate, dipropylene glycol dimethacrylate, polypropylene glycol dimethacrylate, and 2,2-bis(4-methacryloxydiethoxyphenyl)propane; trimethacrylate compounds such as trimethylolpropane trimethacrylate and trimethylolethane trimethacrylate; methylenebisacrylamide, divinylbenzene, and the like can be given. These vinyl polymers may be either a homopolymer or a copolymer of two or more monomers.

To form the polymer part (C) of the magnetic particles in this embodiment, the polymerizable monomers preferably include monomers containing a carboxylic acid group, epoxy group, and the like. These groups are required for causing avidins (discussed later) to chemically bond to the particles. As examples of the monomer having a carboxylic acid group, methacrylic acid, acrylic acid, itaconic acid, beta-methacryloyloxyethyl hydrogenphthalate, beta-acryloyloxyethyl hydrogenphthalate, and the like can be given. As examples of the monomer having an epoxy group, glycidyl methacrylate and the like can be given. In forming the polymer part (C), other mono-functional monomers (b) may be present together with the polymer part (C).

The same polymerization initiator and emulsifier described in connection with the formation of the polymer part (B) can be used for forming the polymer part (C). It is desirable that the polymer part (B) be swelled with a water-insoluble organic solvent prior to formation of the polymer part (C). As examples of such a water-insoluble organic solvent, hydrocarbon solvents such as pentane, hexane, cyclohexane, petroleum ether, petroleum benzine, isooctane, octane, benzene, toluene, and xylene; halogenated compounds such as chloroform, 1,2-dichloroethane, 1,2-dibromoethane, 1,1,2-trichloro-1,2,2-trifluoroethane, chlorobenzene, bromobenzene, and o-dichlorobenzene; and the like can be given.

1.5 Contact of organic solvent (S) with polymer part (B)

As the method of causing the organic solvent (S) to come into contact with the polymer part (B), the following methods can be given, for example. First, after covering the surfaces of the magnetic matrix particles (A) with the polymer part (B) and polymer part (C) according to an above-mentioned process, the particles are magnetically separated from water. The supernatant water is removed. Then, residual water is removed by replacing with a solvent which is freely miscible with water. This solvent is replaced with the organic solvent (S), in which the particles are held. It is preferable to dissolve out the polymer part (B) from the particles in this manner. If necessary, the particles are washed several times with the organic solvent (S). Next, the organic solvent (S) containing the particles is replaced with a solvent which is freely miscible with water. The particles are further washed several times with water to obtain an aqueous dispersion of magnetic particles having porous surfaces of this embodiment. A surface with higher porosity can be obtained by increasing the temperature of the organic solvent (S) when the polymer part (B) is caused to come into contact with the organic solvent (S).

1.6 Application

The magnetic particles of this embodiment can be used in a wide variety of field such as a biochemical field and fields of coating materials, paper, electrophotography, cosmetics, medical supplies, agricultural chemicals, foods, and catalysts.

The main application of the magnetic particles of this embodiment is a biochemical carrier with low non-specific adsorptivity. In addition, the particles of this embodiment can be used as particles for binding biotins by causing a substance having biotin (later described biotins or biotin derivatives) binding sites to be immobilized on the surfaces of the magnetic particles obtained using a polyfunctional monomer (c) having a carboxyl group.

As examples of a substance having biotin binding sites, avidin and derivatives of avidin (hereinafter referred to from time to time collectively as "avidins") such as streptavidin can be given. These biotin-binding particles can reduce a non-specific adsorptivity and increase the amount of binding biotins. As the method of causing avidin or streptavidin to be immobilized on the magnetic particles of this embodiment, a known method described in JP-A-2001-158800, for example, can be used. For example, when the carboxyl group is exposed on the surfaces of the magnetic particles of this embodiment, avidins can be immobilized on the surfaces of the magnetic particles by means of amide bonds formed by causing the amino group in the molecule of avidins to react with the carboxyl group in the presence of a dehydration condensing agent of a water-soluble carbodiimide and the like. In this method, the dehydration condensing agent may be previously reacted with the carboxyl group exposed on the surfaces of the magnetic particles of this embodiment, followed by the addition of avidins to be reacted.

Because the biotin-binding particles of this embodiment have avidins immobilized on the surfaces, the particles can certainly combine with the target substance, for example, nucleic acid, protein, etc, labeled with biotin. In addition, since the avidins can be immobilized on the particles of this embodiment by chemical bonding with a carboxyl group by using a polyfunctional monomer (c) having the carboxyl group as the polymer part (C) when coating the particles with a polymer, there are almost no restrictions to the operating conditions. Therefore, the biotin-binding particles of this embodiment are useful as a diagnostic carrier, bacteria separation carrier, cell separation carrier, nucleic acid separation-purification carrier, protein separation-purification carrier, immobilized enzyme carrier, drug delivery carrier, and the like.

In the biotin-binding particles of this embodiment, protein or oligonucleotide modified with biotin or biotin derivatives (biotin or biotin derivatives are hereinafter referred to from time to time as "biotins") may be immobilized on the surfaces of the particles through avidins.

More specifically, nucleic acid-immobilized particles can be obtained by immobilizing oligonucleotide modified with biotins on the surfaces of the biotin-binding particles of this embodiment. Here, the length of oligonucleotide immobilized, in terms of the number of bases, is from 10 to 100, and preferably from 15 to 70. The oligonucleotide immobilized may be either a single-stranded DNA or a double-stranded DNA, or may be RNA. Such a nucleic acid can be prepared using a commercially available nucleic acid synthesizer. Such nucleic acid-immobilized particles can be used in genetic engineering, including gene diagnosis. Specifically, mRNA can be directly recovered from a cell solution using the nucleic acid immobilized particles of this embodiment combined with oligo-dT, for example. If a nucleic acid having a base sequence complementary with an HIV virus nucleic acid is used as the immobilized nucleic acid, for example, it is possible to collect HIV virus from an *in vivo* sample and to detect HIV virus nucleic acid using the DNA probe method or the PCR method.

In addition, protein-immobilized particles carrying a physiologically active protein thereon can be obtained by immobilizing a physiologically active protein modified with biotins on the surfaces of the biotin-binding particles of this embodiment. As specific examples of such a physiologically active protein, a common protein having physiological activity or its complex compound such as immunoglobulin, various enzyme, and the like can be given. Specifically, antibody-bonded particles and enzyme-bonded particles obtained from the biotin-binding particles of this embodiment can be used for diagnosis and research.

As the method of bonding biotins modified with protein or oligonucleotide with the avidins immobilized on the surfaces of the biotin-binding particles of this embodiment, known methods for bonding biotins with avidins are applicable. For example, particles in which protein or oligonucleotide is immobilized can be obtained by mixing the biotin-binding particles of this embodiment with a protein or oligonucleotide modified with biotins in a phosphate buffer solution containing or not containing 1M sodium chloride at room temperature for 10 minutes to one hour, and removing unreacted protein or unreacted oligonucleotide by solid-liquid separation. It is needless to mention that biotins can be combined with the avidins immobilized on the surfaces of the biotin-binding particles of this embodiment.

As the biotin derivative, for example, biotin-epsilon-N-lysine, biocytin hydrazide, 2-iminobiotin, amino or sulhydril derivatives of biotinyl-epsilon-N-aminocaproic acid-N-hydroxysuccinimide ester, sulfosuccinimide iminobiotin, biotin bromoacetyl hydrazide, p-diazobenzoyl biotin, 3-(N-maleinimidepropionyl) biotin, and the like can be sued.

The method of modifying protein or oligonucleotide with biotins includes, for example, (i) a method of modifying protein with biotins by reacting an ester of biotin and N-hydroxyimide (e.g. biotin-N'-hydroxysuccinimide) with an amino group of the protein molecule, and (ii) a method of modifying a 5'-terminal of oligonucleotide with biotin, comprising bonding phthalimide triethylene glycol to the 5'-terminal of the oligonucleotide, followed by hydrolysis using ammonium hydroxide to form a primary amino group, and bonding the amino group to biotin-N-hydroxysuccinimide, for example. However, the method is not limited to these methods, but various known methods are applicable. It is possible to modify the 3'-terminal of an oligonucleotide with biotins using an appropriate method.

When using the magnetic particles of this embodiment as carrier particles for a diagnostic agent, the following applications can be given, for example: quantitative and qualitative determination of an antibody or antigen utilizing a turbidity change of a solution due to passive agglutination based on an antigen-antibody reaction in which an antigen or antibody of a protein or the like is bonded to the magnetic particles of this embodiment; collection and concentration of viruses, bacteria, cells, chemical compounds such as hormones and dioxin acting as an antigen, by causing an antibody to bond to the magnetic particles of this embodiment and then causing the antigen to bond to the antibody; collection and detection of nucleic acid by bonding a nucleic acid analog such as DNA to the nucleic acid utilizing hybridization, in which the nucleic acid analog such as DNA is bonded with coated magnetic particles of this embodiment; collection and detection of a chemical compound such as a protein or dye bonded to nucleic acid by bonding the protein or dye to the nucleic acid analog; collection and detection of molecules having a biotin or avidin by combining the biotin or avidin with a biotin or avidin bonded to the magnetic particles of this embodiment; use of the magnetic particles of this embodiment bonded with an antibody or antigen as a carrier for enzyme immunity determination utilizing a colorimetric method or a chemical luminescence method; and the like. Conventional diagnostic items utilizing a 96-well plate and the like as a carrier can be replaced with an automatic analyzer using magnetism in which the magnetic particles of this embodiment are used. As the objective substance of diagnosis, hormones such as a protein of biological origin, luteinizing hormone, and thyroid stimulating hormone; various cancer cells; marker proteins such as a prostate gland-specific marker, bladder cancer marker, and the like; viruses such as a hepatitis B virus, hepatitis C virus, and simple herpesvirus; bacteria such as gonococci and MRSA; fungi such as *Candida albicans* and *Cryptococcus;* protozoans and parasites such as toxoplasma; proteins and nucleic acids which are the components of these viruses, bacteria, fungi, protozoans, and parasites; environmental pollutants such as dioxin; chemical compounds used as medical supplies such as an antibiotic and antiepileptic drug; and the like can be given.

The application of the magnetic particles of this embodiment is not limited to the carrier use for biochemistry, but can be used in various fields mentioned above.

2. Examples

The present invention will now be described in more detail by way of examples, which should not be construed as limiting the present invention.

2.1 Evaluation method

The magnetic particles obtained in the examples and comparative examples were evaluated by the following methods. In addition, unless otherwise described, the particle size was measured by the following methods.

2.1.1 Evaluation of biotin bonding amount

In this example, the amount of biotins bonded was evaluated as the bonded amount of biochemical substances.
After dispersing 2 mg of biotin-binding particles obtained in the examples and comparative examples in 1.0 ml of water, 4,000 pmol of a fluorescent biotin (Lucifer yellow cadaverine biotin-X, dipotassium salt) was added and the mixture was mixed at 37°C by inversion for 15 minutes. Next, the particles were separated by a magnetic force and the fluorescence intensity of the supernatant liquid was measured using a spectrofluorophotometer ("PF-777" manufactured by JASCO) to determine the concentration of the fluorescent biotin before the reaction. The amount of unreacted fluorescent biotin was calculated from the determined concentration and the volume of the solution. Furthermore, the amount of bonded biotins (pmol/mg) was determined by determining the difference between the amount of the unreacted fluorescent biotins and the amount of the fluorescent biotin solution (4000 pmol) before binding with the biotin-binding particles (before binding) and dividing the difference by the weight of the particles.
That is, the amount of biotins bonded to the magnetic particles obtained in each example or comparative example was calculated using the following formula (1).

$$\text{Amount of bonded biotins (pmol/mg)} = \{(\text{amount of fluorescence-labeled biotins before bonding (pmol)}) - (\text{amount of unreacted fluorescence-labeled biotins (pmol)})\}/(\text{weight of magnetic particles (mg)}) \qquad (1)$$

The biotin-binding particles used in the examples were prepared by reacting magnetic particles having carboxyl groups on the surfaces with 1-ethyl-3-dimethylaminopropylcarbodiimide hydrochloride which is a condensing agent, and adding strcptavidin to chemically bond the strcptavidin to the reacted and activated magnetic particles.

2.1.2. Particle size

The particle size of particles with a diameter of 1 μm or more was measured using a laser diffraction particle size distribution analyzer ("SALD-200V" manufactured by Shimadzu Corp.). The particle size of particles with a diameter of less than 1 μm was measured using a laser dispersion diffraction particle size distribution analyzer ("LS 13 320" manufactured by Beckmann Coulter).

2.2 Preparation Example 1 (preparation of magnetic matrix particles (A))

Styrene/divinylbenzene (95/5) copolymer particles (average particle size: 1.5 μm) were prepared referring to the polymerization method described in JP-A-7-238105. After the polymerization, particles were removed by centrifugation, washed with water, dried, and ground. The resulting particles are herein referred to as core particles (1).
Acetone was added to an oily magnetic fluid ("EXP series" manufactured by Ferrotec Corp.) to obtain particles as precipitate. The particles were dried to obtain ferrite-based magnetic fine particles with hydrophobized surfaces (average primary particle diameter: 0.02 μm).
Next, 15 g of core particles (1) were sufficiently mixed with 15 g of the hydrophobized magnetic fine particles using a mixer. The mixture was processed using a hybridization system ("NHS-0" manufactured by Nara Machinery Co., Ltd.) at a peripheral speed of the stirring blade of 100 m/sec (16,200 rpm) for 5 minutes to obtain magnetic matrix particles (A) with an average particle diameter of 2.0 μm.

23 Example 1

2.3.1. Preparation of magnetic particles having porous surfaces

A 1-liter separable flask was charged with 750 g of an aqueous solution containing 0.5 wt% of sodium dodecylbenzenesulfonate and 0.5 wt% of a nonionic emulsifier ("Emulgcn 150" manufactured by Kao Corp.), followed by the addition of 30 g of magnetic matrix particles (A), which were dispersed using a homogenizer. The dispersion was heated to 60°C. A pre-emulsion, prepared by adding 15 g of cyclohexyl methacrylate, 0.075 g of tert-dodecanethiol, and 0-75 g of tert-butyl peroxy-2-ethylhaxanate ("Perbutyl O" manufactured by Nippon Oil & Fats Co., Ltd.) to and dispersing in 50 g of an aqueous solution containing 0.5 wt% of sodium dodecylbenzenesulfonate and 0.5 wt% of a nonionic emulsifier ("Emulgen 150" manufactured by Kao Corp.), was added dropwise to the above 1-liter separable flask over two hours, while controlling the temperature at 60°C (formation of polymer part (B)).

After cooling the reaction solution to room temperature, a pre-emulsion, prepared by adding 3 g of isooctane and 0.375 g of tert-butyl peroxy-2-ethylhaxanate ("Perbutyl O" manufactured by Nippon Oil & Fats Co., Ltd.) to and dispersing in 50 g of an aqueous solution containing 0.5 wt% of sodium dodecylbenzenesulfonate and 0.5 wt% of a nonionic emulsifier ("Emulgen 150" manufactured by Kao Corp.), was added to the above 1-liter separable flask, followed by stirring at room temperature for 15 hours. A pre-emulsion, prepared by adding 3.75 g of methacrylic acid and 7.50 g of ethylene glycol dimethacrylate to and dispersing in 50 g of an aqueous solution containing 0.5 wt% of sodium dodecylbenzenesulfonate and 0.5 wt% of a nonionic emulsifier ("Emulgen 150" manufactured by Kao Corp.), was added to the above 1-liter separable flask in which the contents were stirred at room temperature for 15 hours. The mixture was further stirred for two hours at room temperature. The 1-liter separable flask was heated to 80°C to continue the polymerization for a further two hours before termination of the reaction (formation of polymer part (C)). The aqueous dispersion of the resulting magnetic particles was purified by means of a magnetic force and gravity precipitation to obtain an aqueous dispersion of magnetic particles with 1% solid component concentration.

To remove water from the aqueous dispersion of the magnetic particles, the aqueous dispersion was washed twice with 1 liter of acetone. The particles were then dispersed in 1 liter of acetone and the mixture was stirred for two hours, thereby causing the polymer part (B) to come into contact with acetone (organic solvent (S)) (contact of the organic solvent (S) with polymer part (B)). In this manner, the polymer part (B) was dissolved out from the particles using acetone. The particles were washed twice with 1 liter of acetone and then with water to remove acetone, thereby obtaining the magnetic particles of Example 1. An SEM photograph of the magnetic particles of Example 1 is shown in Figure 1. As shown in Figure 1, the magnetic particles of Example 1 were confirmed to have porous surfaces. The average number particle size of the magnetic particles of Example 1 was 2.3 $\mu$m.

2.3.2 Preparation of biotin-binding particles

Next, 0.1 ml of a 0.1 mM HCl solution in which 5 mg of 1-ethyl-3-dimethylaminopropylcarbodiimide hydrochloride (manufactured by Dojindo Chemical Institute, Inc.) was dissolved was added to 1 ml of an aqueous dispersion of the magnetic particles with a solid component concentration of 1 % and the mixture was stirred by rotation at room temperature for two hours. After the addition of 0.1 ml of 0.1 mM HCl solution in which 1 mg of streptavidin (manufactured by Sigma-Aldrich Co.) was dissolved, the mixture was stirred by rotation at room temperature for eight hours to prepare biotin-binding particles with streptavidin immobilized on the surfaces. The suspension containing the biotin-binding particles was processed by magnetic separation to obtain a solid component (biotin-binding particles). A procedure of adding a phosphate buffer solution containing 0.1% bovine serum albumin (PBS, 0.1% BSA/PBS, pH = 7.2) to the solid, followed by magnetic separation, was repeated three times to remove unreacted streptavidin. The suspension of biotin-binding particles with a solid component concentration of 1 % was obtained by dispersing the biotin-binding particles in a phosphate buffer solution containing 0.1 % bovine serum albumin (PBS, 0.1% BSA/PBS, pH = 7.2) to provide a solid component concentration of 1 % . The amount of biotin bonded to the biotin-binding particles was evaluated using the suspension thus obtained. The results are shown in Table 1. The number average number particle size of the biotin-binding particles of Example 1 was 2.3 $\mu$m.

2.4 Example 2

2.4.1. Preparation of magnetic particles having porous surfaces

A 1-liter separable flask was charged with 750 g of an aqueous solution containing 0.5 wt% of sodium dodecylbenzenesulfonate and 0.5 wt% of a nonionic emulsifier ("Emulgen 150" manufactured by Kao Corp.), followed by the addition of 30 g of magnetic particles (A), which were dispersed using a homogenizer. The dispersion was heated to 60°C. A pre-emulsion, prepared by adding 7.5 g of cyclohexyl methacrylate, 0.75 g of ethylene glycol dimethacrylate, and 0.38 g of tert-butyl peroxy-2-ethylhaxanate ("Perbutyl O" manufactured by Nippon Oil & Fats Co., Ltd.) to and dispersing in 75 g of an aqueous solution containing 0.5 wt% of sodium dodecylbenzenesulfonate and 0.5 wt% of a nonionic emulsifier ("Emulgen 150" manufactured by Kao Corp.), was added dropwise to the above-mentioned 1-liter separable flask over two hours, while controlling the temperature at 60°C. After increasing the temperature to 80°C, the mixture was reacted for two hours (formation of polymer part on magnetic matrix particles (A)).

After cooling the reaction solution to 60°C, a pre-emulsion, prepared by adding 15 g of cyclohexyl methacrylate, 0.075 g of tert-dodocanethiol, and 0.75 g of tert-butyl peroxy-2-ethylhaxanate ("Perbutyl O" manufactured by Nippon Oil & Fats Co., Ltd.) to and dispersing in 75 g of an aqueous solution containing 0.5 wt% of sodium dodecylbenzenesulfonate and 0.5 wt% of a nonionic emulsifier ("Emulgen 150" manufactured by Kao Corp.), was added dropwise to the 1-liter separable flask over two hours, while controlling the temperature at 60°C. After increasing the temperature to 80°C, the mixture was reacted for two hours (formation of polymer part (B)).

Then, the reaction mixture was cooled to room temperature and a pre-emulsion prepared by using 0.375 g of tert-butyl peroxy-2-ethylhaxanate ("Perbutyl O" manufactured by Nippon Oil & Fats Co., Ltd.) was added to the 1-liter separable flask. The mixture was stirred at room temperature for 15 hours.

Next, a pre-emulsion, prepared by adding 3.75 g of methacrylic acid and 7.50 g of ethylene glycol dimethacrylate to and dispersing in 50 g of an aqueous solution containing 0.5 wt% of sodium dodecylbenzenesulfonate and 0.5 wt% of a nonionic emulsifier ("Emulgen 150" manufactured by Kao Corp.), was added to the above 1-liter separable flask. The mixture was stirred for two hours at room temperature. The 1-liter separable flask was heated to 80°C to continue the polymerization for a further two hours before termination of the reaction (formation of polymer part (C)). The aqueous dispersion of the resulting magnetic particles was purified by means of a magnetic force and gravity precipitation to obtain an aqueous dispersion of magnetic particles with 1 % solid component concentration. The average number particle size of the magnetic particles of Example 2 was 2.1 $\mu$m.

Next, the polymer part (B) was caused to come into contact with an organic solvent (S) (acetone) in the same manner as in Example 1 to obtain an aqueous dispersion of magnetic particles of Example 2. Inspection using SEM confirmed porous surfaces on the magnetic particles in the aqueous dispersion.

2.4.2 Preparation of biotin-binding particles

The amount of biotin bonded to the magnetic particles obtained in Example 2 was evaluated in the same manner as in Example 1. The results are shown in Table 1. The average number particle size of the biotin-binding particles of Example 2 was 2.1 $\mu$m.

2.5 Comparative Example 1

Magnetic particles of Comparative Example 1 were obtained in the same manner as in Example 1, except for omitting the polymerization of the polymer part (B) (cyclohexyl methacrylate), but performing only the polymerization of the polymer part (C). Inspection of the magnetic particles of the Comparative Example 1 using SEM confirmed that the surfaces were not porous. The amount of biotin bonded to the magnetic particles obtained in Comparative Example 1 was evaluated in the same manner as in Example 1. The results are shown in Table 1.

2.6 Comparative Example 2 Magnetic particles before causing the polymer part (B) to come into contact with the organic solvent (S) (acetone) in Example 1 were obtained as the magnetic particles of Comparative Example 2. Inspection of the magnetic particles of the Comparative Example 2 using SEM confirmed that the surfaces were not porous. The amount of biotin bonded to the magnetic particles obtained in Comparative Example 2 was evaluated in the same manner as in Example 1. The results are shown in Table 1.

Table 1 shows the amount of biotins bonded to the magnetic particles of Examples and Comparative Examples.

TABLE I

|  | Magnetic matrix particle (A) | Polymer part (B) | Polymer part (C) | Contact of organic solvent (S) with polymer part (B) | Amount of bonded biotin (pmol/mg) |
|---|---|---|---|---|---|
| Example 1 | Matrix particle coating layer was not formed | CHMA/t-DS | Methacrylic acid /EDMA | Yes | 1,200 |
| Example 2 | Matrix particle coating layer (CHMA/EDMA layer) was formed | CHMA/t-DS | Methacrylic acid / EDMA | Yes | 1,100 |

(continued)

|  | Magnetic matrix particle (A) | Polymer part (B) | Polymer part (C) | Contact of organic solvent (S) with polymer part (B) | Amount of bonded biotin (pmol/mg) |
|---|---|---|---|---|---|
| Comparative Example 1 | Matrix particle coating layer was not formed | None | Methacrylic acid /EDMA | Yes | 250 |
| Comparative Example 2 | Matrix particle coating layer was not formed | CHMA/t-DS | Methacrylic acid /EDMA | No | 300 |
| CHMA: Cyclohexyl methacrylate EDMA: Ethylene glycol dimethacrylate t-DS: tert-dodecanethiol | | | | | |

The SEM photograph of Figure 1 confirms that the magnetic particles of Example 1 have porous surfaces.

In addition, the magnetic particles of Examples 1 and 2, which are obtained by forming a polymer part (B) soluble in an organic solvent (S) and a polymer part (C) insoluble in the organic solvent (S) on the surfaces of magnetic matrix particles (A) and causing the polymer part (B) to come into contact with the organic solvent (S), have porous surfaces and, therefore, can bond biotins in a larger amount than the magnetic particles of Comparative Examples 1 and 2 as indicated in Table 1. In this manner, the magnetic particles of Examples 1 and 2 were confirmed to exhibit excellent magnetic separation capability and capability of binding a large amount of biochemical substances.

Although only some embodiments of the invention have been described in detail above, those skilled in the art will readily appreciate that many modifications are possible in the embodiments without departing from the novel teachings and advantages of this invention. Accordingly, all such modifications are intended to be included within the scope of this invention.

Magnetic particles having porous surfaces are obtained by forming a polymer part (B) soluble in an organic solvent (S) and a polymer part (C) insoluble in the organic solvent (S) on surfaces of magnetic matrix particles (A) and causing the polymer part (B) to come into contact with the organic solvent (S).

**Claims**

1. Magnetic particles having porous surfaces, the magnetic particles being obtained by forming a polymer part (B) soluble in an organic solvent (S) and a polymer part (C) insoluble in the organic solvent (S) on surfaces of magnetic matrix particles (A) and causing the polymer part (B) to come into contact with the organic solvent (S).

2. The magnetic particles having porous surfaces as defined in claim 1, wherein the polymer part (B) is formed by radical polymerization of a mono-functional monomer (b) in an aqueous medium in the presence of the magnetic matrix particles (A).

3. The magnetic particles having porous surfaces as defined in claim 1 or 2, wherein the polymer part (C) is formed by radical polymerization of a poly-functional monomer (c) in an aqueous medium in the presence of the magnetic matrix particles (A).

4. The magnetic particles having porous surfaces as defined in any one of claims 1 to 3, wherein the magnetic matrix particles (A) contains:

    non-magnetic nuclear particles $(A_1)$; and
    magnetic fine particles $(A_2)$ located on the surfaces of the non-magnetic nuclear particles $(A_1)$.

5. The magnetic particles having porous surfaces as defined in claim 4, further containing a matrix particle coating layer $(A_3)$ which covers the non-magnetic nuclear particles $(A_1)$ and the magnetic fine particles $(A_2)$ wherein the matrix particle coating layer $(A_3)$ comprises the polymer part (C), and the surface of the matrix particle coating layer $(A_3)$ is the porous surface.

6. A carrier for use with a biochemical substance, comprising the magnetic particles having porous surfaces as defined in any one of claims 1 to 5.

7. Biotin-binding particles, comprising a substance having biotin binding sites immobilized on the surfaces of the magnetic particles having porous surfaces as defined in any one of claims 1 to 5.

8. A method of producing magnetic particles having porous surfaces, comprising:

Forming a polymer part (B) soluble in an organic solvent (S) and a polymer part (C) insoluble in the organic solvent (S) on surfaces of magnetic matrix particles (A); and
causing the polymer part (B) to come into contact with the organic solvent (S).

9. The method of preparing the magnetic particles having porous surfaces as defined in claim 8,
wherein the step of forming the polymer part (B) includes radically polymerizing a mono-functional monomer (b) in an aqueous medium in the presence of the magnetic matrix particles (A).

10. The method of preparing the magnetic particles having porous surfaces as defined in claim 8 or 9,
wherein the step of forming the polymer part (C) includes radically polymerizing a poly-functional monomer (c) in an aqueous medium in the presence of the magnetic matrix particles (A).


**Patentansprüche**

1. Magnetische Teilchen mit porösen Oberflächen, wobei die magnetischen Teilchen erhalten sind durch Bilden eines Polymerteils (B), der in einem organischen Lösungsmittel (S) löslich ist, und eines Polymerteils (C), der in einem organischen Lösungsmittel (S) unlöslich ist, auf Oberflächen von magnetischen Matrixteilchen (A) und Bewirken, dass der Polymerteil (B) mit dem organischen Lösungsmittel (S) in Kontakt tritt.

2. Magnetische Teilchen mit porösen Oberflächen nach Anspruch 1, wobei der Polymerteil (B) durch radikalische Polymerisation eines monofunktionalen Monomers (b) in einem wässrigen Medium in der Anwesenheit der magnetischen Matrixteilchen (A) gebildet ist.

3. Magnetische Teilchen mit porösen Oberflächen nach Anspruch 1 oder 2, wobei der Polymerteil (C) durch radikalische Polymerisation eines polyfunktionalen Monomers (c) in einem wässrigen Medium in der Anwesenheit der magnetischen Matrixteilchen (A) gebildet ist.

4. Magnetische Teilchen mit porösen Oberflächen nach einem der Ansprüche 1 bis 3, wobei die magnetischen Matrixteilchen (A) enthalten:

nichtmagnetische Kernteilchen $(A_1)$; und
magnetische Feinteilchen $(A_2)$, die auf den Oberflächen der nichtmagnetischen Kernteilchen $(A_1)$ lokalisiert sind.

5. Magnetische Teilchen mit porösen Oberflächen nach Anspruch 4, ferner enthaltend eine Matrixteilchenüberzugsschicht $(A_3)$, welche die nichtmagnetischen Kernteilchen $(A_1)$ und die magnetischen Feinteilchen $(A_2)$ bedeckt, wobei die Matrixteilchenüberzugsschicht $(A_3)$ den Polymerteil (C) umfasst und die Oberfläche der Matrixteilchenüberzugsschicht $(A_3)$ die poröse Oberfläche ist.

6. Träger zur Verwendung mit einer biochemischen Substanz, der die magnetischen Teilchen mit porösen Oberflächen nach einem der Ansprüche 1 bis 5 umfasst.

7. Biotin bindende Teilchen, die eine Substanz mit Biotinbindungsstellen immobilisiert auf den Oberflächen der magnetischen Teilchen mit porösen Oberflächen nach einem der Ansprüche 1 bis 5 umfassen.

8. Verfahren zur Herstellung magnetischer Teilchen mit porösen Oberflächen, umfassend:

Bilden eines Polymerteils (B), der in einem organischen Lösungsmittel (S) löslich ist, und eines Polymerteils (C), der in dem organischen Lösungsmittel (S) unlöslich ist, auf Oberflächen von magnetischen Matrixteilchen (A); und

Bewirken, dass der Polymerteil (B) in Kontakt mit dem organischen Lösungsmittel (S) tritt.

**9.** Verfahren zur Herstellung der magnetischen Teilchen mit porösen Oberflächen nach Anspruch 8, wobei der Schritt des Bildens des Polymerteils (B) radikalische Polymerisation eines monofunktionalen Monomers (b) in einem wässrigen Medium in der Anwesenheit der magnetischen Matrixteilchen (A) beinhaltet.

**10.** Verfahren zur Herstellung der magnetischen Teilchen mit porösen Oberflächen nach Anspruch 8 oder 9, wobei der Schritt des Bildens des Polymerteils (C) radikalische Polymerisation eines polyfunktionalen Monomers (c) in einem wässrigen Medium in der Anwesenheit der magnetischen Matrixteilchen (A) beinhaltet.

**Revendications**

**1.** Particules magnétiques ayant des surfaces poreuses, les particules magnétiques étant obtenues en formant une partie polymère (B) soluble dans un solvant organique (S) et une partie polymère (C) insoluble dans le solvant organique (S) sur des surfaces de particules matrice magnétiques (A) et en faisant en sorte que la partie polymère (B) vienne en contact avec le solvant organique (S).

**2.** Particules magnétiques ayant des surfaces poreuses selon la revendication 1, dans lesquelles la partie polymère (B) est formée par polymérisation radicalaire d'un monomère monofonctionnel (b) dans un milieu aqueux en présence des particules matrice magnétiques (A).

**3.** Particules magnétiques ayant des surfaces poreuses selon la revendication 1 ou 2, dans lesquelles la partie polymère (C) est formée par polymérisation radicalaire d'un monomère polyfonctionnel (c) dans un milieu aqueux en présence des particules matrice magnétiques (A).

**4.** Particules magnétiques ayant des surfaces poreuses selon l'une quelconque des revendications 1 à 3, dans lesquelles les particules matrice magnétiques (A) contiennent :

des particules nucléaires non magnétiques ($A_1$) ; et
des particules fines magnétiques ($A_2$) situées sur les surfaces des particules nucléaires non magnétiques ($A_1$).

**5.** Particules magnétiques ayant des surfaces poreuses selon la revendication 4, contenant en outre une couche de revêtement de particules matrice ($A_3$) qui recouvre les particules nucléaires non magnétiques ($A_1$) et les particules fines magnétiques ($A_2$),
dans lesquelles la couche de revêtement de particules matrice ($A_3$) comprend la partie polymère (C), et la surface de la couche de revêtement de particules matrice ($A_3$) est la surface poreuse.

**6.** Support destiné à une utilisation avec une substance biochimique, comprenant les particules magnétiques ayant des surfaces poreuses selon l'une quelconque des revendications 1 à 5.

**7.** Particules se liant à la biotine, comprenant une substance ayant des sites de liaison à la biotine immobilisés sur les surfaces des particules magnétiques ayant des surfaces poreuses selon l'une quelconque des revendications 1 à 5.

**8.** Procédé de production de particules magnétiques ayant des surfaces poreuses, comprenant :

la formation d'une partie polymère (B) soluble dans un solvant organique (S) et d'une partie polymère (C) insoluble dans le solvant organique (S) sur des surfaces de particules matrice magnétiques (A) ; et
la mise en contact de la partie polymère (B) avec le solvant organique (S).

**9.** Procédé de production de particules magnétiques ayant des surfaces poreuses selon la revendication 8, dans lequel l'étape de formation de la partie polymère (B) inclut la polymérisation radicalaire d'un monomère monofonctionnel (b) dans un milieu aqueux en présence des particules matrice magnétiques (A).

**10.** Procédé de production de particules magnétiques ayant des surfaces poreuses selon la revendication 8 ou 9, dans lequel l'étape de formation de la partie polymère (C) inclut la polymérisation radicalaire d'un monomère polyfonctionnel (c) dans un milieu aqueux en présence des particules matrice magnétiques (A).

# FIG.1

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 4774265 A
- JP 5197212 A
- JP 7027279 B
- US 5814687 A
- JP 57024369 B
- JP 61215602 A
- JP 61215603 A
- JP 61215604 A
- JP 2001158800 A
- JP 7238105 A

**Non-patent literature cited in the description**

- *J. Polym. Sci., Polymer Letter Ed.,* 1963, vol. 21, 937